Europäisches Patentamt

European Patent Office

Office européen des brevets

(19)

(11) Veröffentlichungsnummer: **0 194 326 B1**

## EUROPÄISCHE PATENTSCHRIFT

(12)

(45) Veröffentlichungstag der Patentschrift: **07.08.91**

(51) Int. Cl.5: **A61F 2/38**

(21) Anmeldenummer: **85102629.4**

(22) Anmeldetag: **08.03.85**

(54) **Kniegelenk-Endoprothese.**

(43) Veröffentlichungstag der Anmeldung:
**17.09.86 Patentblatt 86/38**

(45) Bekanntmachung des Hinweises auf die
Patenterteilung:
**07.08.91 Patentblatt 91/32**

(84) Benannte Vertragsstaaten:
**AT BE CH DE FR GB IT LI NL SE**

(56) Entgegenhaltungen:
EP-A- 0 069 683      CH-A- 555 671
DE-A- 2 452 412      DE-A- 2 802 568
DE-A- 3 339 102      DE-B- 2 549 819
US-A- 3 837 009

(73) Patentinhaber: **orthoplant Endoprothetik
GmbH
Leerkämpe 12
W-2800 Bremen 66(DE)**

(72) Erfinder: **Schlegel, Karl Friedrich, Prof.
Dr.med.
Virochwstrasse 66
W-4300 Essen 1(DE)**
Erfinder: **Schelhas, Klaus-Dieter
Sempertstrasse 3
W-2000 Hamburg 60(DE)**

(74) Vertreter: **Eisenführ, Speiser & Strasse
Martinistrasse 24
W-2800 Bremen 1(DE)**

## Beschreibung

Die Erfindung betrifft eine Kniegelenk-Endoprothese, deren Femurteil am unteren Ende eines Verankerungselements zwei konvex gekrümmte Kondylenschalen, und deren Tibiateil am oberen Ende eines Tibiaschaftes ein Tibiaplateau aufweist, dessen beide Tibiastützflächen zum Abstüzen der Kondylenschalen entsprechend konkav gekrümmt sind, wobei zwischen den beiden Tibiastützflächen im dorsalen Bereich des Tibiaplateaus ein Tibiahöcker dorsal-ventral ausgerichtet ist und sich nach oben in einen Spalt zwischen den Kondylenschalen erstreckt, und mit einem Querstift am Femurteil, der durch ein Auge des Tibiahöckers medial-lateral hindurchgeführt ist.

Aus der CH-A-555 671 ist eine derartige Kniegelenk-Endoprothese bekannt, bei welcher das Auge im Tibiahöcker als gekrümmtes Langloch ausgebildet ist, dessen Breite dem Querschnitt des femuralen Querstifts entspricht, um dem femuralen Querstift eine Zwangsführung zu verleihen, so daß das Femurteil nur eine vom femuralen Querstift und dem tibialen Langloch definierte Zwangsbewegung ausführen kann. Roll- und Gleitbewegungen, welche bei der natürlichen Kniebewegung zwischen den natürlichen Kondylen und den natürlichen Tibiastützflächen stattfinden, lassen sich daher nicht verwirklichen. Darüber hinaus gestattet diese bekannte Endoprothese auch keine axiale Rotation des Tibia, die bei dem natürlichen Kniegelenk mit wachsender Flexion möglich ist. Aufgrund der von der natürlichen Kniebewegung abweichenden Zwangsbewegung zwischen Femurteil und Tibiateil wird die Verankerung der Endoprothese oftmals unerwünscht stark belastet.

Aus der DE 33 39 102 ist eine Kniegelenk-Endoprothese bekannt, bei der das Tibiateil und das Femurteil mittels eines separaten Gelenkteils verbunden sind, welches im Tibiateil in einer dorsal aufwärts gerichteten Bohrung bei Flexion des Kniegelenks aufwärts verschiebbar ist, wodurch die Roll- und Gleitbewegung der Kondylenschale auf dem Tibiaplateau ermöglicht wird. Nachteilig bei dieser bekannten Endoprothese ist es, daß die Strecklage durch einen harten Anschlag begrenzt wird, und daß eine axiale Relativrotation zwischen Tibia- und Femurteil unbegrenzt möglich ist.

Aus der DE-OS 31 19 841 ist eine Kniegelenk-Endoprothese bekannt, bei der das Femurteil um eine lateral-medial ausgerichtete Rotationsachse rotiert, die ihrerseits um einen vertikalen Tibiazapfen drehbar ist und dadurch eine Nachbildung der um die Tibiaachse möglichen Rotation des Tibia ermöglicht. Aufgrund der Zwangsbewegung um die medial-laterale Rotationsachse läßt sich die natürliche Roll- und Gleitbewegung der Kondylen nicht nachbilden, die Zwangsbewegungen belasten vielmehr die Verankerungen. Darüber hinaus nimmt die Rotationsmechanik dieser Endoprothese relativ viel Raum ein, so daß ein vergleichsweise großer Bereich der Kondylen reseziert werden muß.

Aus der US 3 837 009 ist eine Kniegelenk-Endoprothese bekannt, deren Tibiateil ein ovales Auge enthält, das einen am Femurteil befestigten Querstift umschließt. Bei einer Beugung des Gelenks läuft der Querstift in eine untere Ausweitung des Auges hinein, die beiden Gelenkteile haben dann relativ zueinander ein vorgegebenes Bewegungsspiel. Beim Übergang in die Strecklage läuft der Querstift dagegen in eine obere Verengung des Auges und wird dort in der Strecklage zwangsgeführt.

Aufgabe der Erfindung ist es demgegenüber, eine Kniegelenk-Endoprothese der eingangs genannten Art derart weiterzubilden, daß die natürlichen Bewegungsmöglichkeiten des Knies unter der Wirkung des bei der Implantation erhaltenen natürlichen Bänderapparates gut nachgebildet werden.

Diese Aufgabe wird erfindungsgemäß bei der Kniegelenk-Endoprothese der eingangs genannten Art dadurch gelöst, daß das Auge kreisförmig oder oval ausgebildet ist und so groß bemessen ist, daß jeder durch den Zentralbereich des Auges verlaufende Durchmesserstrahl eine Länge besitzt, die mindestens dem doppelten Durchmesser des femuralen Querstiftes entspricht, und daß der Querstift in der Strecklage der Endoprothese vom Augenrand beabstandet ist.

Die Vorteile der Erfindung liegen insbesondere darin, daß zwischen Kondylenschalen und Tibiastützflächen ein Gleitlager ausgebildet ist, welches bei der relativen Flexion von Femur und Tibia eine Rollbewegung der Kondylenschalen auf dem Tibiaplateau ermöglicht, der zwischen den Extremstellungen maximaler Beugung eine Gleitbewegung überlagert ist.

Da das Auge gegenüber dem Durchmesser des Querstiftes vergleichsweise groß ist, bleibt der durch das Auge hindurchgeführte Querstift bei allen natürlichen Bewegungen und Stellungen der Kniegelenk-Teile vom Augenrand beabstandet. Die natürlichen Bewegungsabläufe können ohne Zwangsführung und ohne Anschläge durchgeführt werden und werden dabei von dem natürlichen Bänderapparat kontrolliert, der bei der Operation möglichst weitgehend erhalten bleiben soll. Gleichwohl stellt der im Auge gefangene Stift sicher, daß bei starken Querkräften, die auf Femur oder Tibia wirken können, keine Luxation der Gelenkteile stattfindet; nur unter starken Querkräften findet eine relative Verschiebung zwischen Tibiateil und Femurteil statt, diese Bewegung wird jedoch dadurch beendet, daß der Querstift gegen den Augenrand anschlägt.

In einer bevorzugten Ausführungsform der Erfindung verläuft der Querstift in dem Spalt zwischen den Kondylenschalen und ist an diesen befestigt. Der Tibiahöcker besitzt dann eine Einführöffnung im Augenrand, welche das Einführen des Querstiftes in das Auge ermöglicht. Um das Einführen besonders einfach zu machen, ist die Einführöffnung an der ventralen Kante des Tibiahökkers angeordnet, der dorsale Augenrand ist geschlossen, um einen zuverlässigen Anschlag für den Querstift zu bilden.

Besonders bevorzugt besitzt die Einführöffnung Sperrmittel, so z.B. einen Riegel o.dgl., die ein Einführen des Querstiftes in das Auge zulassen, welche jedoch den Querstift am Verlassen des Auges wirksam hindern. Gemäß einer bevorzugten Ausführungsform der Erfindung sind die Sperrmittel durch eine geschickte Formgebung der Einführöffnung verwirklicht. Wird nämlich die Einführöffnung zur Vertikalebene des Tibiahöckers ausreichend geneigt, so ist die Einführung des femuralen Querstiftes nur unter einer vorgegebenen künstlichen Neigung gegenüber dem Tibiaplateau möglich, die bei der natürlichen Kniebewegung nicht eingenommen werden kann. Der bei der Implantation schließlich in das Auge eingeschobene Querstift kann dann - nach dem Aufsetzen der Kondylenschalen auf die Tibiastützflächen und nach richtiger Positionierung des Bandapparates am Ende der Operation - nicht mehr aus dem Auge austreten.

Besonders bevorzugt besitzen die beiden Kondylenschalen dorsal je eine den interkondyloiden Spalt begrenzende Seitenwand. Der Querstift ist an den beiden Seitenwänden befestigt und erstreckt sich lateral-medial durch den Spalt. Der Abstand zwischen den beiden Seitenwänden ist dabei so bemessen, daß zwischen dem Tibiahöcker und den Seitenwänden je ein ausreichender Zwischenraum verbleibt.

Die dorsalen Seitenwände der Kondylenschalen sind - in einer Ausführungsform der Erfindung - am ventralen Ende des interkondyloiden Spaltes mittels einer Querwand miteinander verbunden. Die Seitenwände können in lateral-medial verlaufende dorsale Abschnitte der Kondylenstützflächen übergehen.

Vorteilhafte Weiterbildungen der Erfindung sind durch die Merkmale der Unteransprüche gekennzeichnet.

Im folgenden wird ein Ausführungsbeispiel der Erfindung anhand der Zeichnung näher erläutert.

Es zeigen:

Fig. 1     einen Längsschnitt durch eine erfindungsgemässe Kniegelenk-Endoprothese nebst Knochen im implantierten Zustand in Strecklage, wobei das Tibiateil - im Gegensatz zu der Darstellung gemäss den Fig. 2 und 3 - nicht geschnitten ist;

Fig. 2     eine Fig. 1 entsprechende Teildarstellung in überstreckter Position, wobei - im Gegensatz zu Fig. 1 - das Tibiateil kurz vor seiner Mittellinie geschnitten ist, wie dieses in Fig. 9 mit einer Schnitt-linie II, III-II, III angedeutet ist;

Fig. 3     eine Fig. 2 entsprechende Darstellung bei extremer Flexion:

Fig. 4     eine Seitenansicht des Femurteils in Richtung des Pfeiles IV in Fig. 5 gesehen;

Fig. 5     eine Vorderansicht auf das Femurteil in Richtung des Pfeiles V in Fig. 4;

Fig. 6     eine Draufsicht von oben auf das Femurteil gemäss den Fig. 4 und 5 in Richtung des Pfeiles VI in Fig. 5 gesehen;

Fig. 7     eine Seitenansicht des Femurteils gemäss den Fig. 4 bis 6 in Richtung des Pfeiles VII in Fig. 4 gesehen;

Fig. 8     eine Draufsicht auf das Tibiateil in Richtung des Pfeiles VIII in Fig. 9 gesehen;

Fig. 9     eine Seitenansicht des Tibiateils gemäss Fig. 8 in Richtung des Pfeiles IX in Fig. 8 gesehen;

Fig. 10    eine Seitenansicht des die Kondylen enthaltenden unteren Abschnittes eines Femurknochens in Richtung des Pfeiles X in Fig. 11 gesehen; und

Fig. 11    eine Seitenansicht des unteren Abschnittes des Femurknochens in Richtung des Pfeiles XI in Fig. 10 gesehen, d.h. also in Richtung auf die kleinere Kondyle.

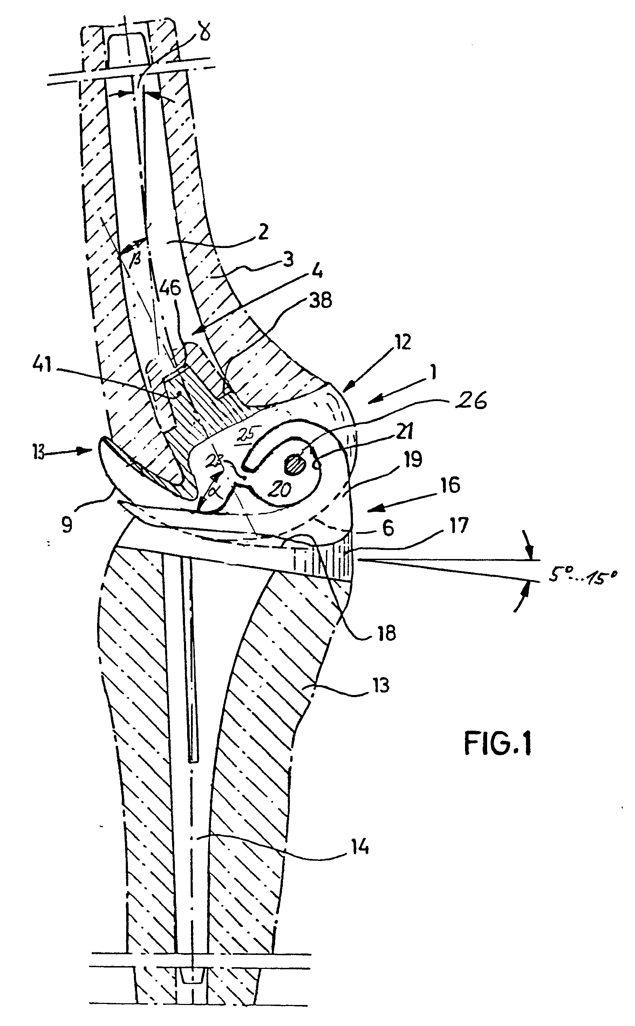

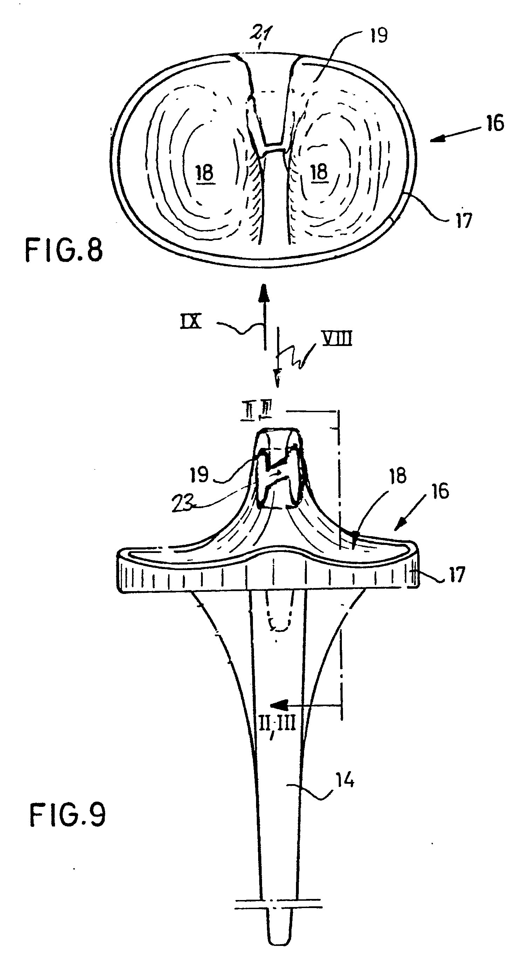

Gemäss den Fig. 1 bis 9 besitzt die Kniegelenk-Endoprothese 1 ein mittels eines Verankerungselements 2 am Femur 3 verankerbares Femurteil 4, das an seinem unteren Ende zwei konvex gekrümmte Kondylenschalen 11, 12 besitzt. Die nach unten weisenden Flächen der Kondylenschalen 11, 12 stellen entsprechend konvex gekrümmte Kondylenstützflächen 8, 9 dar.

Ausserdem ist die Endoprothese 1 mit einem am Tibia 13 mittels eines Tibiaschaftes 14 verankerbaren Tibiateils 16 versehen, das an seinem oberen Ende ein Tibiaplateau 17 aufweist, dessen beide Tibiastützflächen 18 entsprechend der grössten Krümmung der Kondylenschalen konkav gekrümmt sind. Das Tibiaplateau 17 besteht aus einer mit dem Tibiaschaft 14 fest verbundenen metallischen Tibiaplatte 17', die eine Tibiaeinlage 17" aus Kunststoff enthält, in welche die Tibiastützflächen 18 eingeformt sind. Das Tibiaplateau 17 fällt nach hinten leicht ab und schliesst dadurch in ventral-dorsaler Richtung mit der Achse des Tibiaschaftes 14 einen Winkel von etwa 75 bis 85° ein.

Die beiden Tibiastützflächen 18 verlaufen dorsalventral. Zwischen den beiden Tibiastützflächen 18 ist im dorsalen Bereiche des Tibiaplateaus 17 ein Tibiahöcker 19 ebenfalls dorsal-ventral ausgerichtet und erstreckt sich nach oben in einen Spalt 25 zwischen den Kondylenschalen 11, 12. Am Femurteil 4 ist ein Querstift 26 befestigt, der den Tibiahöcker 19 medial-lateral durchdringt. Die Kondylenstützflächen bilden zusammen mit den Tibiastützflächen ein Roll- und Gleitlager, welches die natürliche Gleit- und Rollbewegung des Kniegelenks bei seiner Flexion nachbildet.

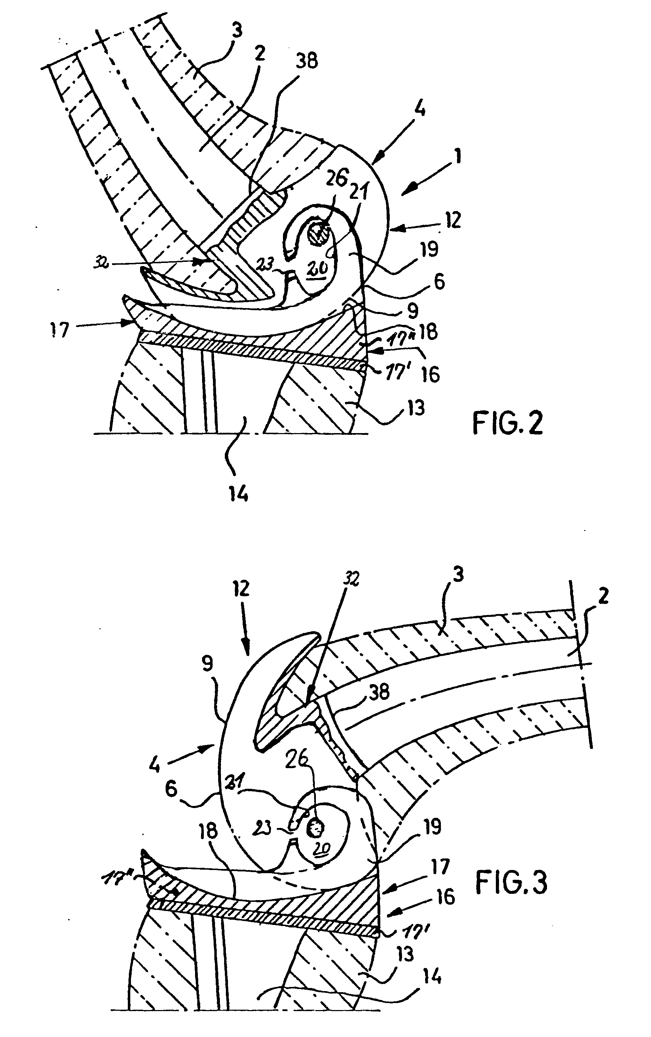

Wie insbesondere den Fig. 1 bis 3 entnehmbar ist, besitzt der Tibiahöcker 19 ein Auge 20, welches den am Femurteil 4 befestigten Querstift 26 aufnimmt. In der dargestellten Ausführungsform ist das Auge 20 kreisförmig oder oval ausgebildet und so groß bemessen, daß der Querstift (26) im Auge (20) frei beweglich ist und in der in Fig. 1 dargestellen Strecklage sowie in der in Fig. 3 dargestellten starken Flexionslage - und in allen Zwischenlagen zwischen den beiden Stellungen - den Augenrand 21 des tibialen Auges 20 nicht berührt. Die Bewegung läuft daher als Roll- und Gleitbewegung ohne Zwangsführung des Querstiftes 26 und ohne mechanischen Anschlag ab so dass auch Kipp- und Rotationsbewegungen der Prothesenteile möglich sind. Bei einer Überstreckung des Kniegelenks oder bei starken Querkräften zwischen Tibia- und Femurteil läuft der Querstift 26 gegen den Augenrand 21, wodurch die Flexion oder die Querverschiebung von Tibia oder Femur relativ zueinander beendet wird.

Die beiden Kondylenschalen 11, 12 sind in ihrer Grösse und ihrer Form den beiden Kondylen des natürlichen Femurs angepasst und besitzen eine im wesentlichen gleichbleibende geringe Wandstärke, die eine Resektion von Teilen der Kondylen überflüssig macht. Dorsal besitzen die Kondylenschalen 11, 12 je eine Seitenwand 28, 29, welche den interkondyloiden Spalt 25 begrenzt. Die Seitenwände gehen dorsal in eine Rückwand über, die integral in die Kondylenstützflächen 8, 9 übergeht. Die Seitenwände 28, 29 der Kondylenschalen sind am ventralen Ende des interkondyloiden Spaltes 25 mittels einer Querwand miteinander verbunden, um die Stabilität der Seitenwände zu erhöhen und um ausserdem eine Begrenzungswand zu den sich daran anschliessenden Kondylen zu schaffen und dadurch die Auge/Querstift-Verbindung vom Knochengewebe zu trennen.

Der Querstift 26 verläuft quer durch den interkondyloiden Spalt 25 und ist an beiden Seitenwänden 28, 29 der Kondylenschalen 11, 12 befestigt. Um - bei dieser Ausführungsform - den Querstift in das Auge 20 einzuführen, ist im Augenrand 21 eine Einführöffnung 23 vorgesehen, die mit Sperrmitteln verschlossen sein kann, welche das Einführen des Querstiftes 26 in die Augenöffnung zulassen, welche jedoch den Querstift an einem Verlassen des Auges 20 hindern.

Um ein einfaches Einführen des femuralen Querstiftes 26 in das tibiale Auge 20 während der Implantation der Endoprothese 1 zu ermöglichen, befindet sich die Einführöffnung 23 im ventralen Abschnitt des Augenrandes 21. Der obere und der dorsale Abschnitt des Augenrandes 21 erhält dadurch die Form eines einstückigen Hakens, welcher die Bewegung des Querstiftes 26 bei unnatürlichen Knie-Beanspruchungen zuverlässig begrenzt.

Anstelle von Sperrmitteln, z.B. Schliessfedern od.dgl., lässt sich der Einführöffnung 23 eine solche Gestalt geben, dass während der Implantation durch eine vom Operateur künstlich erzeugte Neigungsstellung der femurale Querstift durch die Einführöffnung 23 in das Auge 20 hineingleiten kann, dass der Querstift jedoch das Auge 20 bei allen im einoperierten Zustand möglichen Relativlagen nicht mehr verlassen kann. Zu diesem Zweck verläuft die Einführöffnung 23 zur dorsalventralen Vertikalebene des Tibiahöckers 19 ausreichend geneigt, so dass das Einführen des femuralen Querstiftes 26 nur unter einer vorgegebenen künstlich erzeugten Neigung des Femurteils relativ zum Tibiaplateau 17 möglich wird.

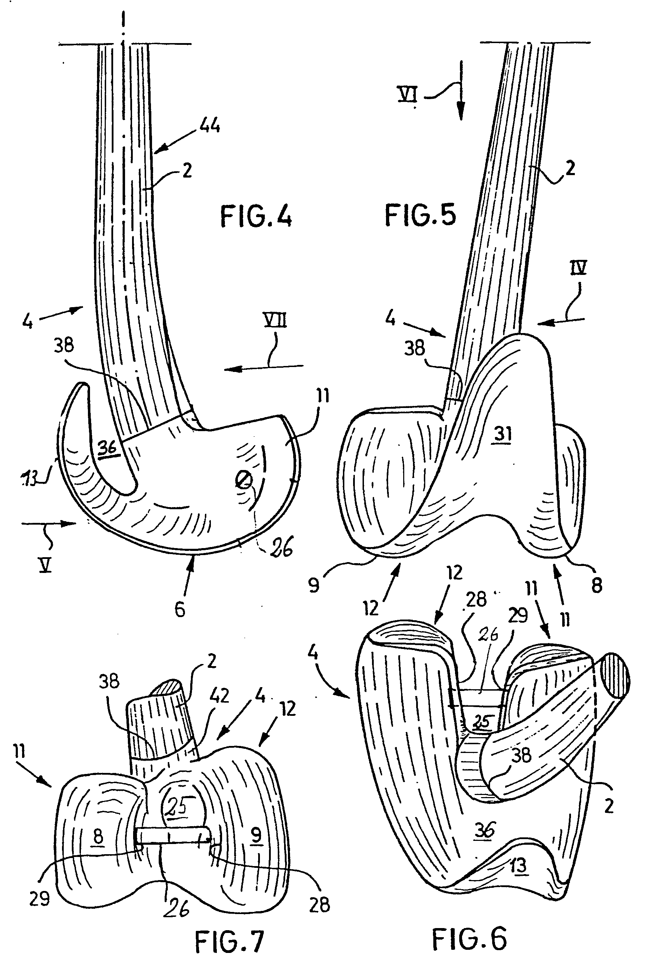

Die Kondylenschalen 11, 12 gehen ventral im Anschluss an den interkondyloiden Spalt 25 ineinander über und münden in ventraler Richtung in eine konvex nach oben gekrümmte Patellazunge 13. Der Zwischenraum 36, welcher durch die Innenfläche der Patellazunge 13, die Innenfläche der Kondylenschalen 11, 12 und das Verankerungselement 2 gebildet ist, bildet eine Hinterschneidung, wie insbesondere aus Fig. 4 erkennbar ist. Das Verankerungselement 2 ist im Bereich der Hinterschneidung eine Teilfuge 38 zwischen dem einstückig an die Kondylenschalen 11, 12 über dem ventralen Ende des Spaltes 25 angeformten Konuszapfen 41 und dem auf dem Konuszapfen 41 mit einer entsprechenden Konusausnehmung 46 aufgesteckten Femurschaft 42. Diese zweiteilige Ausführungsform des Verankerungselementes 2 dient dazu, die Implantation der Endoprothese zu erleichtern.

Die Achse des Konuszapfens 41 ist nach vorn geneigt und schliesst gegenüber den Stützflächen 8, 9 der Kondylenschalen 11, 12 etwa einen Winkel $\alpha$ zwischen 40 und 75° ein. Der Femurschaft 42 besitzt an seinem unteren Fusspunkt einen gekrümmten Abschnitt 43, der ventral konvex gekrümmt ist und eine Länge 1 von etwa 3 bis 7 cm besitzt und an seinem oberen Ende in einen geraden freien Endabschnitt 44 übergeht. Der Endabschnitt 44 des Femurschaftes 42 ist gegenüber der Achse des Konuszapfens - d.h. gegenüber der Fusspunkt-Neigung - um .etwa weitere 20 bis 400 aufwärts geneigt,($\beta$), so dass die Achse des freien Endabschnitts in Strecklage relativ zur Achse des Tibiaschaftes 14 einen Winkel $\gamma$ von etwa 10 bis 20° einschliesst.

Wie bereits erwähnt, sind die Kondylenschalen 11, 12 der Form der natürlichen Kondylen angepasst und daher unterschiedlich gross. Die Kondylenstützflächen 8, 9 besitzen eine unterschiedliche Krümmung, und zwar ist der Krümmungsradius im dorsalen Abschnitt der kleineren Kondylenschale 11 relativ klein und geht zur Unterseite 6 hin weitgehend stetig in einen grösseren Krümmungsradius über. Der Krümmungsradius der grösseren Kondylenschale 12 ist sowohl in seinem dorsalen als auch in seinem unteren Bereich grösser als derjenige der kleineren Kondylenschale 11. Die grössere Kondylenschale verläuft ein vorgegebenes Maß tiefer wie die kleinere Kondylenschale 11.

Da die Krümmung der Tibiastützflächen 18 im wesentlichen gleich der grössten Krümmung der Kondylenstützflächen 8, 9 an deren Unterseite 6 ist, liegen letztere in Strecklage (Fig. 1) und insbesondere in überstreckter Stellung (Fig. 2) formschlüssig und damit gegen eine Rotation gehalten in den Tibiastützflächenabschnitten 18', 18''. Bei steigender Flexion kommt es indes dazu, dass die Abschnitte mit kleinerem Krümmungsradius der Kondylenstützflächen 8, 9 mit den Tibiastützflächen 18 in Eingriff kommen, so dass auf diese Weise sukzessiv und selbsttätig gesteuert bei steigender Flexion eine entsprechend ansteigende Rotationsmöglichkeit zwischen Femur und Schienbein ermöglicht ist und die Kniegelenk-Endoprothese 1 mithin insgesamt praktisch sämtliche Bewegungs- und Arretierungsverhältnisse simuliert, wie diese unter natürlichen Gegebenheiten vorhanden sind.

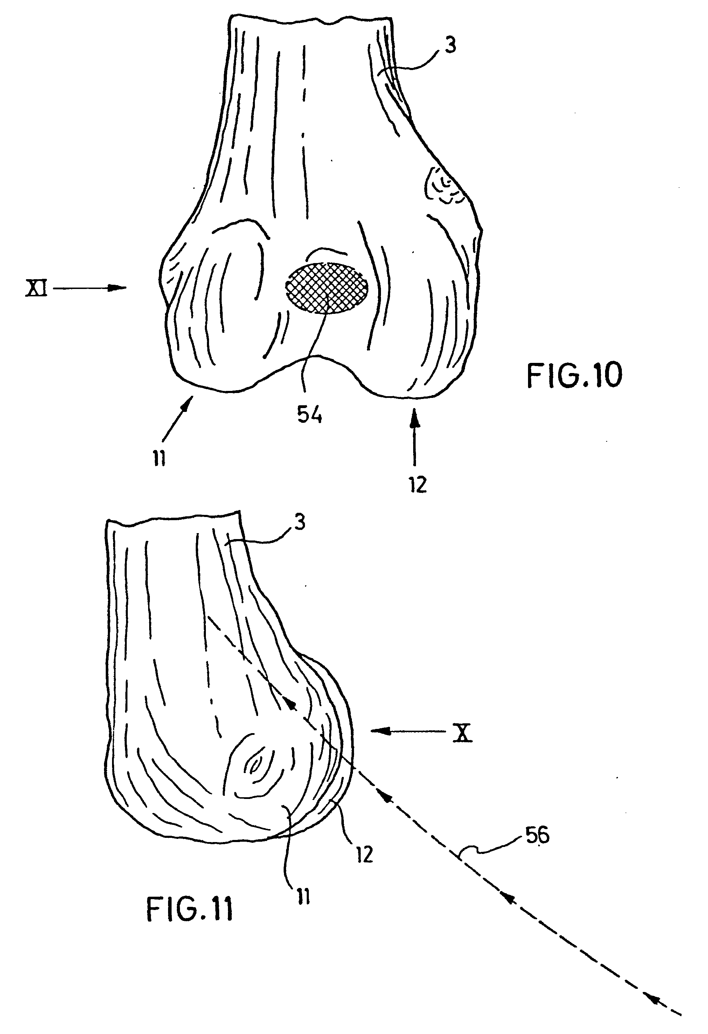

Die Implantation der Endoprothese 1 ist ausserordentlich gewebeschonend und belässt im Gegensatz zu bisher bekannten Operationsverfahren die wesentlichen Teile des Femurs bzw. dessen Kondylen ohne Schwächung. Da die Endoprothese 1 keinen eigentlichen femuralen Block besitzt, der im natürlichen Femurgewebe verankert werden muss und demgemäss ein entsprechend grosses interkondyläres Fenster erfordert und während bei den bekannten Kniegelenkprothesen weiterhin der grössere Kondylus 12 durch spanabhebende Bearbeitung auf das Maß des kleineren Kondylus 11 gebracht wird (wobei häufig mehr als 10 mm abgetragen werden müssen), ist eine derartige Resektion bei der erfindungsgemässen Prothese nicht erforderlich. Vielmehr wird bei dieser lediglich zwischen den natürlichen Kondylen 11 und 12 (siehe Fig. 10, 11) an einer Stelle, wo ohnehin kein tragendes Knochengewebe vorhanden ist, nämlich bei den Kreuzbändern, eine dem Femurschaft 42 bzw. dessen Durchmesser von ca. 2 cm entsprechende Bohrung 54 angebracht, wie sie in Fig. 10 mit einer strichpunktierten Ellipse angedeutet und kreuzschraffiert dargestellt ist, und die Prothese wird sodann mit dem freien Endabschnitt des Femurschaftes 42 voran in Richtung der Pfeil-Strichlinie 56 (Fig.11) in die Bohrung 54 eingebracht. Aufgrund der Krümmung des Femurschaftes 42 lässt sich dieser ohne Schwierigkeiten durch die Bohrung 54 in den Markkanal des Femurs 3 einbringen.

Dabei erfolgt die Verankerung, wie bereits oben angedeutet, so, dass zunächst der Femurschaft 42 so weit durch die Bohrung 54 in den Femur eingetrieben wird, dass er noch ein Stückchen aus diesem heraussteht. Sodann wird der untere Endabschnitt 7 des Femurteils 4 angesetzt, d.h. der Konuszapfen 41 des Femurteils 4 wird in die konische Aufnahmeöffnung 45 des Femurschaftes 42 eingesetzt und die Kondylenschalen 11, 12 werden sodann mit einem geeigneten Werkzeug gleichsam um die natürlichen Kondylen herumgelegt, so dass auch die Hinterschneidung an der Patellazunge 13 keinerlei Schwierigkeiten macht. Insgesamt ergibt sich dann schliesslich im Ergebnis gleichsam - zumindest bezüglich des femuralen Teils - eine Art Schalenprothese mit den bekannten Vorteilen einer solchen Konstruktion.

Bezüglich des tibialen Teils sei noch darauf verwiesen, dass hierfür, wie ersichtlich, der Schienbeinknochen an seinem oberen Endabschnitt in der aus Fig. 1 erkennbaren Weise, also unter einem Winkel $\alpha$ von beispielsweise 5°, reseziert wird, und dass sodann das Tibiateil 16 mit dem Tibiaschaft 14 voran in den Markkanal des Schienbeinknochens 13 eingetrieben und dort verankert wird.

## Patentansprüche

1. Kniegelenk-Endoprothese, deren Femurteil (4) am unteren Ende eines Verankerungselements (2) zwei konvex gekrümmte Kondylenschalen (11, 12), und deren Tibiateil (16) am oberen Ende eines Tibiaschaftes (14) ein Tibiaplateau (17) aufweist, dessen beide Tibiastützflächen (18) zum Abstützen der Kondylenschalen (11, 12) entsprechend konkav gekrümmt sind, wobei zwischen den beiden Tibiastützflächen (18) im dorsalen Bereich (21) des Tibiaplateaus (17) ein Tibiahöcker (19) dorsal-ventral ausgerichtet ist und sich nach oben in einen Spalt (25) zwischen den Kondylenschalen erstreckt, und mit einem Querstift (26) am Femurteil (4), der durch ein Auge (20) des Tibiahöckers (19) medial-lateral hindurchgeführt ist, dadurch gekennzeichnet, daß das Auge (20) kreisförmig oder oval ausgebildet und so groß bemessen ist, daß jeder durch den Zentralbereich des Auges (20) verlaufende Durchmesserstrahl eine Länge besitzt, die mindestens dem doppelten Durchmesser des femuralen Querstiftes (26) entspricht, und daß der Querstift in der Strecklage der Endoprothese vom Augenrand beabstandet ist.

2. Kniegelenk-Endoprothese nach Anspruch 1, dadurch gekennzeichnet, daß der Querstift (26) an den beiden Kondylenschalen (11, 12) befestigt ist, und daß der Tibiahöcker (19) eine Einführöffnung (23) im Augenrand (21) zur Einführung des Querstifts (26) aufweist.

3. Kniegelenk-Endoprothese nach Anspruch 2, dadurch gekennzeichnet, daß die Einführöffnung (23) am ventralen Augenrand (21) des Tibiahöckers (19) angeordnet ist.

4. Kniegelenk-Endoprothese nach Anspruch 2, dadurch gekennzeichnet, daß die Einführöffnung (23) an der oberen oder dorsalen Kante des Tibiahöckers (19) angeordnet ist.

5. Kniegelenk-Endoprothese nach einem der Ansprüche 2 bis 4, dadurch gekennzeichnet, daß die Einführöffnung (23) Sperrmittel enthält, die den im Auge (20) eingefangenen Querstift (26) an einem Verlassen des Auges (20) hindern.

6. Kniegelenk-Endoprothese nach einem der Ansprüche 2 bis 5, dadurch gekennzeichnet, daß die Einführöffnung (23) zur Vertikalebene des Tibiahöckers (19) geneigt verläuft und das Einführen des femuralen Querstiftes (26) nur unter einer vorgegebenen Neigung relativ zum Tibiaplateau (17) ermöglicht.

7. Kniegelenk-Endoprothese nach einem der vorstehenden Ansprüche, dadurch gekennzeichnet, daß die Kondylenschalen (11, 12) dorsal je eine den interkondyloiden Spalt (25) begrenzende Seitenwand (28, 29) besitzen, und daß der Querstift (26) sich durch den Spalt (25) erstreckt und an den Seitenwänden (28, 29) befestigt ist.

8. Kniegelenk-Endoprothese nach Anspruch 7, dadurch gekennzeichnet, daß die dorsalen Seitenwände (28, 29) am ventralen Ende des interkondyloiden Spalts (25) mittels einer Querwand (30) miteinander verbunden sind.

**Claims**

1. A kneejoint endoprosthesis of which the femur portion (4) exhibits at the bottom end of an anchoring element (2) two convexly curved condyle shells (11, 12) and the tibia portion (16) exhibits at the top end of tibia shank (14) a tibia plateau (17) of which the two tibia bearing areas (18) for bearing against the condyle shells (11, 12) are concavely curved to correspond, whilst in the dorsal region (21) of the tibia plateau (17) between the two tibia bearing areas (18) a tibia protuberance (19) is aligned dorsally-ventrally and extends upwards into a gap (25) between the condyle shells, and having on the femur portion (4) a crosspin (26) which is passed with medial-to-lateral alignment through an eye (20) in the tibia protuberance (19), characterized in that the eye (20) is made circular or oval and of a size such that every diametral ray running through the central region of the eye (20) has a length which corresponds with at least twice the diameter of the femoral crosspin (26), and that the crosspin is clear of the edge of the eye in the extended position of the endoprosthesis.

2. A kneejoint endoprosthesis as in Claim 1, Characterized in that the crosspin (26) is fastened to the two condyle shells (11, 12), and that the tibia protuberance (19) exhibits an inlet opening (23) in the edge (21) of the eve for introducing the crosspin (26).

3. A kneejoint endoprosthesis as in Claim 2, characterized in that the inlet opening (23) is arranged at the ventral edge (21) of the eye in the tibia protuberance (19).

4. A kneejoint endoprosthesis as in Claim 2, characterized in that the inlet opening (23) is arranged at the upper or dorsal edge of the tibia protuberance (19).

5. A kneejoint endoprosthesis as in one of the Claims 2 to 4, Characterized in that the inlet opening (23) contains blocking means which when the crosspin (26) is caught in the eye (20) prevent it from leaving the eye (20).

6. A kneejoint endoprosthesis as in one of the Claims 2 to 5, characterized in that the inlet opening (23) runs at a slant to the vertical plane of the tibia protuberance (19) and enables introduction of the femoral crosspin (26)only at a predeterimined slant relative to the tibia plateau (17).

7. A kneejoint endoprosthesis as in one of the preceding Claims,

characterized in that each of the condyle shells (11, 12) has dorsally a sidewall (28, 29) bounding the intercondyloid gap (25), and that the crosspin (26) extends through the gap (25) and is fastened to the sidewalls (28, 29).

8. A kneejoint endoprosthesis as in Claim 7, characterized in that the dorsal sidewalls (28, 29) are connected together by means of a crosswall (30) at the ventral end of the intercondyloid gap (25).

**Revendications**

1. Endoprothèse de l'articulation du genou dont la partie fémorale (4) comporte, à l'extrémité inférieure d'un élément d'ancrage (2), deux coques de condyles (11, 12) à courbure convexe, et dont la partie tibiale (16) comporte à l'extrémité supérieure d'une tige de tibia (14), un plateau tibial (17) dont les deux surfaces glénoïdes tibiales (18) destinées à l'appui des coques de condyles (11, 12) présentent une courbure concave appropriée, un pic tibial (19) d'orientation dorsale-ventrale, prévu entre les surfaces glénoïdes tibiales (18) dans la région dorsale (21) du plateau tibial (17), s'étendant vers le haut dans une fente (25) entre les coques de condyles, et une goupille transversale (26) sur la partie fémorale (4) passant de manière médiane-latérale au travers d'un oeil (20) du pic tibial (19), caractérisée en ce que l'oeil (20) est de forme circulaire ou ovale et présente des dimensions telles que chaque faisceau diamétral passant par la zone centrale de l'oeil (20), présente une longueur au moins égale au double du diamètre de la goupille transversale fémorale (26), et que dans la position tendue de l'endoprothèse, la goupille transversale est éloignée du bord de l'oeil.

2. Endoprothèse de l'articulation du genou selon la revendication 1, caractérisée en ce que la goupille transversale (26) est fixée sur les deux coques de condyles (11, 12), et en ce que le pic tibial (19) présente dans le bord (21) de l'oeil, une ouverture d'introduction (23) destinée à l'engagement de la goupille transversale (26).

3. Endoprothèse de l'articulation du genou selon la revendication 2, caractérisée en ce que l'ouverture d'introduction (23) est disposée sur le bord ventral (21) de l'oeil du pic tibial (19).

4. Endoprothèse de l'articulation du genou selon la revendication 2, caractérisée en ce que l'ouverture d'introduction (23) est disposée sur le bord supérieur ou dorsal du pic tibial (19).

5. Endoprothèse de l'articulation du genou selon l'une des revendications 2 à 4, caractérisée en ce que l'ouverture d'introduction (23) comporte des moyens de verrouillage qui empêchent la goupille transversale (26) emprisonnée dans l'oeil (20), de s'échapper de cet oeil (20).

6. Endoprothèse de l'articulation du genou selon l'une des revendications 2 à 5, caractérisée en ce que l'ouverture d'introduction (23) s'étend de manière inclinée par rapport au plan vertical du pic tibial (19) en ne permettant l'engagement de la goupille transversale fémorale (26) que sous une inclinaison prédéfinie, par rapport au plateau tibial (17).

7. Endoprothèse de l'articulation du genou selon l'une des revendications précédentes, caractérisée en ce que chacune des coques de condyles (11, 12) comporte au niveau dorsal, une paroi latérale (28, 29) délimitant la fente intercondylienne (25), et en ce que la goupille transversale (26) s'étend au travers de la fente (25) et est fixée sur les parois latérales (28, 29).

8. Endoprothèse de l'articulation du genou selon la revendication 7, caractérisée en ce que les parois latérales dorsales (28, 29) sont reliées entre-elles à l'extrémité ventrale de la fente intercondylienne (25), au moyen d'une paroi transversale (30).

FIG.1

5°...15°

FIG.2

FIG.3

FIG.4

FIG.5

FIG.7

FIG.6

10

FIG.8

FIG.9

FIG.10

FIG.11